# EUROPEAN PATENT APPLICATION

(11) **EP 2 952 899 A1**
(43) Date of publication of application: **09.12.2015**
(21) Application number: 14171499.8
(22) Date of filing: 06.06.2014
(51) Int. Cl.: G01N 33/68

(54) **NKG2C as prognostic marker in multiple sclerosis**

(71) Applicant: Fundació Institut Mar d'Investigacions Mèdiques (IMIM), 08003 Barcelona (ES)
(72) Inventor: Martínez Rodriguéz, José Enrique, 08003 Barcelona (ES); López-Botet, Miguel, 08003 Barcelona (ES); Munteis Olivas, Elvira, 08003 Barcelona (ES); Roquer Gonzalez, Jaume, 08003 Barcelona (ES); Muntasell Castellvi, Aura, 08003 Barcelona (ES)
(74) Representative: ZBM Patents - Zea, Barlocci & Markvardsen

(57) **Abstract**

The present invention provides an *in vitro* method for determining the clinical prognosis of a subject diagnosed of multiple sclerosis (MS), the method comprising the step of determining the genotype of *NKG2C* gene in an isolated test sample from the subject.

In addition the present invention provides the use of means for determining the genotype of *NKG2C.*

The method of the invention provides valuable information about the clinical progression of MS.

SEQUENCE LISTING

<110>  Fundació Institut Mar d'Investigacions Mèdiques

<120>  NKG2C as prognostic marker in multiple sclerosis

<130>  P3037EP00

<160>  4

<170>  PatentIn version 3.5

<210>  1
<211>  21
<212>  DNA
<213>  Artificial Sequence

<220>
<223>  primer

<400>  1
actcggattt ctatttgatg c                                                21


<210>  2
<211>  22
<212>  DNA
<213>  Artificial Sequence

<220>
<223>  primer

<400>  2
acaagtgatg tataagaaaa ag                                               22


<210>  3
<211>  18
<212>  DNA
<213>  Artificial Sequence

<220>
<223>  primer

<400>  3
cagtgtggat cttcaatg                                                    18


<210>  4
<211>  22
<212>  DNA
<213>  Artificial Sequence

<220>
<223>  primer

<400>  4
tttagtaatt gtgtgcatcc ta                                               22

## Description

The invention relates to a method for the prognosis of multiple sclerosis, more specifically to a method for predicting progression of neurological disability in patients diagnosed with multiple sclerosis.

### BACKGROUND ART

Multiple sclerosis (MS) is a complex immune-mediated demyelinating disease of the central nervous system (CNS) with a heterogeneous and unpredictable clinical evolution, ranging from a relatively benign clinical course to a rapidly disabling disease. MS affects the ability of neurons in the brain and spinal cord to communicate with each other and control body functions. Relapsingremitting MS (RRMS) is the most frequent MS subtype (85%) and is characterized by relapses, attributed to new inflammatory demyelinating lesions on CNS white matter, which leads to periods of neurological disability usually followed by periods of clinical remission. Globally, half of RRMS patients after 15-20 years will develop secondary progressive MS (SPMS) with increasing disability, attributed to axonal damage and neurodegeneration. In a subset of MS patients (10-15%), progression is present at initial stages of the disease and is not related with relapses (primary-progressive MS, PPMS).

Multiple sclerosis is a chronic disease that affects young individuals at their most productive period of life, with striking personal and professional consequences. The incidence and prevalence of MS is increasing in the last years and, nowadays, it is among the most frequent causes of permanent neurological disability in young individuals. At present, the etiology of MS is unknown, but it is thought to result from a combination of genetic and environmental factors.

Currently, MS diagnosis is based on McDonald criteria 2010 (Polman 2011), lacking specific diagnostic tests. The clinical presentation of the disease is supported by the analysis of the cerebrospinal fluid (CSF) and evoked potential (EP) studies of the visual, auditory and somatosensory pathways, as well as by Magnetic Resonance Imaging (MRI) of the brain and spinal cord. These techniques, however, have some disadvantages. For example, the analysis of CSF may detect the presence of oligoclonal bands (OCB) by electrophoresis in up to 75-90% of MS patients, supporting the notion of chronic CNS inflammation. However, OCB may be not detected in all MS patients and, moreover, do not provide accurate clinical information for progression and long-term prognosis. Furthermore, the extraction of CSF involves an invasive lumbar puncture procedure that may cause a major discomfort to the subject with possible side effects such as dizziness and severe headache. Regarding MRI techniques, current predictors of disease evolution based on conventional MRI data have limited value for long-term prognosis, probably related to their limited specificity in characterizing and quantifying the heterogeneous pathological features of MS, in particular the neurodegenerative process.

From a clinical perspective, patients with different MS clinical presentations show different responses to treatment. For instance, RRMS patients are more likely to respond to immunomodulatory therapy than those with a progressive disease course. Thus, predicting the clinical course of MS would also provide information on disease management and selection for different lines of therapies according to disease severity with the aim to delay the begining of progressive stages

Biomarkers are anatomic, physiologic, biochemical or molecular parameters associated with specific disease states. The search for MS biomarkers has been focused on indicators of the general activity of the inflammatory process as well as neurodegenerative aspects of the disease.

There remains an unmet need for a non-invasive and cost-effective technique for prognosing multiple sclerosis disability in MS patients.

### SUMMARY OF THE INVENTION

The present inventors have found in MS patients that analysis of *NKG2C* genotype provides a valuable information regarding the clinical course of MS.

Thus, in a first aspect the present invention provides an *in vitro* method for determining the clinical prognosis of MS in a MS patient, the method comprising the step of determining the genotype of *NKG2C* gene in an isolated test sample from the subject.

Despite considerable advances regarding new immunomodulatory therapies, the long-term prognosis of the disease is currently unpredictable, lacking reliable biomarkers to identify at early stages which patients are going to develop disability and, subsequently, which patients can get a higher benefit for second- or third-line therapies. Therefore, the analysis of NKG2C as prognostic marker in MS may provide valuable information in MS patients to select those patients with a higher risk of progression that could get an early benefit once instaured second- or third-line therapies.

In a second aspect the present invention provides the use of means for determining the genotype of *NKG2C* in an isolated sample for performing the method of the first aspect of the invention.

### BRIEF DESCRIPTION OF THE DRAWINGS

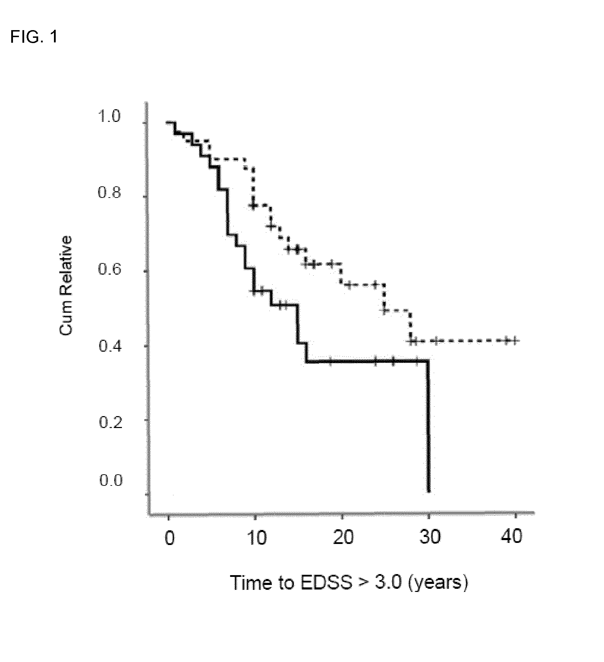

FIG. 1 represents a Kaplan-Meier-survival analysis for an EDSS>3.0 classifying MS patients according to NKG2C zygosity. Dotted line represents homozygosity; continous line represents hemizygosity. HR 1.930, CI95% 1.006-3.701, p 0.048. EDSS: Expanded Disability Status Scale.

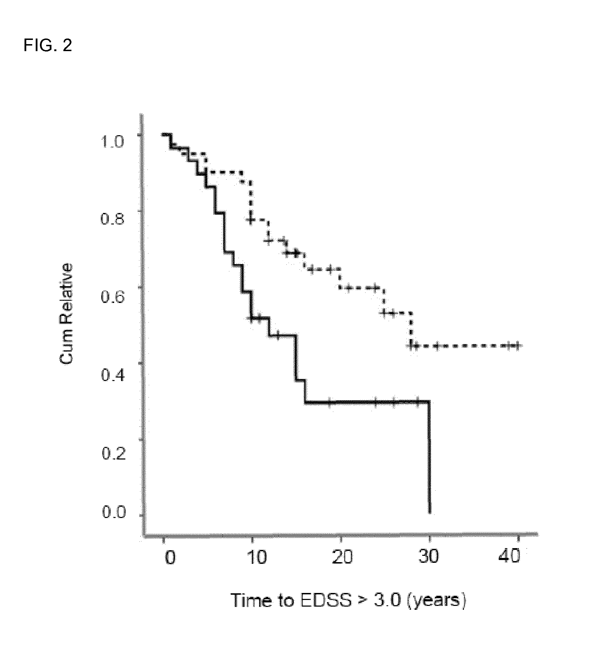

FIG. 2 represents a Kaplan-Meier-survival analysis for an EDSS>3.0 classifying MS patients according to NKG2C zygosity and NKG2C bright pattern. Dotted line represents homozygosity or a NKG2C bright surface expression; continuous line represents hemizygosity. HR 2.348, CI95% 1.200-4.594, p 0.013. EDSS: Expanded Disability Status Scale.

### DETAILED DESCRIPTION OF THE INVENTION

The present invention provides a prognostic method based on the genotype analysis of *NKG2C.*

According to the present invention, "determining the clinical prognosis" is understood as giving an opinion as to the future condition of the patient (clinical (physical and cognitive) disability) after a determined period of time. The clinical prognosis can be performed in recently diagnosed patients or after the first flare-up, as well as at any time of the disease's course. The condition of the patient can be defined based on the symptoms of multiple sclerosis, including a reduced capacity for controlling small movements, reduced attention span, reduced coordination, reduced discerning capacity, reduced memory, depression, difficulty in speaking or understanding language, dizziness, double vision, eye discomfort, facial pain, fatigue, loss of balance, problems with movement that are slowly progressive and begin in the legs, muscle atrophy, muscle spasms (especially in the legs), muscle spasticity (uncontrollable spasm of muscle groups), numbling or abnormal sensation in any area, pain in the arms and legs, paralysis of one or both arms or legs, dysarthria, tingling sensation, shaking in one or both arms or legs, uncontrollable and rapid eye movements, increased urinary frequency, difficulty in urinating, urinary urgency, urinary incontinence, vertigo, loss of vision, walk/gait abnormaliy and weakness in one or both arms or legs.

NKG2C, also known as killer cell lectin-like receptor, subfamily C, member 2, is a type II transmembrane protein with extracellular C-type lectin domain. This protein heterodimerizes with CD94 to form a surface receptor that recognizes HLA-E as ligand. The NKG2C/CD94 complex provides an activating signal due to its association with the ITAM-containing DAP12 adapter protein. These complexes are expressed on NK and T cell subsets. NKG2C gene and protein sequences are available in published databases (Homo sapiens NKG2C gene, GenBank accesion number AJ001684.1, Version AJ001684.1 GI: 2989858, PRI 14-NOV-2006; Protein accesion CAA04922, version CAA04922.1. GI:2980859, PRI 14-NOV-2006).

In the present invention the sentence "determining the genotype" is synonym of "genotyping" and is to be understood as the process of elucidating the genotype in an individual with a biological assay. Also known as a genotypic assay, techniques include PCR, DNA fragment analysis, allele specific oligonucleotide (ASO) probes, DNA sequencing, and nucleic acid hybridization to DNA microarrays or beads. Several common genotyping techniques include restriction fragment length polymorphism (RFLP), terminal restriction fragment length polymorphism (t-RFLP), amplified fragment length polymorphism (AFLP), and multiplex ligation-dependent probe amplification (MLPA). All of them being well-known for the skilled person in the art.

In one embodiment, the subject is a human cytomegalovirus (HCMV) seropositive MS patient.

According to the present invention, "human cytomegalovirus (HCMV) seropositive MS patient" is a subject which has been diagnosed of MS and is infected by HCMV.

It is well-known in the state of the art that herpesviruses are common pathogens that establish lifelong infections with periodical reactivations that have been associated with MS. Most seroepidemiological studies point out to a pathogenic role of the Epstein-Barr virus (EBV), a gamma-herpesvirus that infects 90% of the adult population and is almost universally present in MS patients. In contrast to the putative pathogenic role of EBV in MS, recent reports have related human cytomegalovirus (HCMV) infection with a lower risk to develop MS. However, whether HCMV has a protective effect in MS and which mechanisms are involved remains to be determined. HCMV infection promotes a redistribution of the Natural Killer (NK) cell compartment whose hallmark is an expansion of a mature NK-cell subset displaying high surface levels of the CD94/NKG2C activating receptor (NKG2C bright), with additional distinctive phenotypic and functional features. Beyond its known pathogenic effects in immunocompromised individuals, the lifelong coexistence with HCMV may have a multifaceted impact on human health, and might contribute to the pathogenesis of chronic inflammatory disorders. In this regard, most previous studies analyzing herperviruses influence on MS are based on the analysis of pathogen-specific IgG titers as a marker of the humoral immune responses, which does not reflect the complexity of the individual host-pathogen relationship. Morevoer, these studies do not provide information about the risk of progression in MS.

As it is shown below, genotyping *NKG2C* gene may provide valuable information about clinical progression in HCMV seropositive MS patients. In addition, from the experimental data provided below, the present inventors believe that the protective effect of HCMV in MS patients might be associated with an expansion of peripheral NKG2C+ NK cells, being the analysis of NK cell immunophenotype and genotype putative predictors of MS clinical disease evolution.

Therefore, in one embodiment of the first aspect of the invention, the genotype of the *NKG2C* gene is hemizygote and it is indicative that the subject has a bad prognosis.

As it has been explained above, MS is defined in terms of physical signs and symptoms. It is unpredictable from such clinical symptomatology whether the disease can progress (i.e., irreversible clinical disability). This lack of information hindered the task of the physician, who has to change the treatment either from detecting changes in such physical signs/symptoms or once there has been a relapse event.

The present invention means a great advance in the field of MS prognosis because it provides valuable information about the progression of the disease just determining the genotype of NKG2C in MS patients. If the physician determines that the subject is hemizygote, a more accurate therapeutic protocol could be designed based on second- and third-line therapies. The implementation of such therapeutic protocol in an early stage could significantly slow down the progression of the disease and related accumulating disability as well as improve health-related quality of life.

In the present invention "hemizygote" is to be understood as that human chromosome 12 includes uniquely one gene copy codifying for NKG2C.

In the present invention "bad prognosis" is to be understood as that MS likely will progress to irreversible stages of clinical disability.

The present inventors have also found that when, in addition to *NKG2C* genotype, the NKG2C protein expression by NK cells is determined, more accurate information can be obtained about whether and how the disease will progress.

Thus, in one embodiment, the method of the first aspect of the invention further comprises the step of determining the surface expression level of NKG2C protein by NK cells.

The information concerning how fast the progression is expected to be can be obtained comparing the level obtained from the test sample with reference values taken from healthy controls.

In another embodiment, the determination of the surface expression level of NKG2C protein is performed prior, after or simultaneously to the step of genotyping the *NKG2C* gene.

The expression "simultaneously", when referred to perform both the genotyping step and the determination of the surface expression level of NKG2C protein in NK cells, means that both steps are performed at the same time but separately, each one making use of the necessary reagents and conditions to obtain the intended information.

In one embodiment, the determination of the protein surface expression is performed after the step of genotyping.

In another embodiment, the *in vitro* method comprises:
(a) determining the surface expression level of NKG2C protein in NK cells in the test sample of the subject; and
(b) comparing the level determined in step (a) with a reference NKG2C control value,
wherein if the level determined in step (a) is lower than the reference control value, it is indicative that the subject has a higher risk for bad prognosis.

In the present invention, the term "reference control value" referred is to be understood as a predefined value of molecular marker NKG2C, which is derived from the levels of said molecular marker in a sample or group of samples. The samples are taken from a group of healthy controls. This value is used as a threshold to determine the risk of progressing of the disease and even how the progression will be. The skilled person in the art, making use of the general knowledge, is able to choose the subject or group of subjects more adequate for obtaining the reference control level for the methods of the present invention.

Methods for obtaining the reference value from the group of subjects selected are well-known in the state of the art (Burtis C. A. et al., 2008, Chapter 14, section "Statistical Treatment of Reference Values"). In a particular case "reference control level" is a cut-off value defined by means of a conventional ROC analysis (Receiver Operating Characteristic analysis). As the skilled person will appreciate, optimal cut-off value will be defined according to the particular applications of the prognostic method: purpose, target population for the prognosis, and balance between specificity and sensibility, among others.

At the moment of determining the reference control level, the nature of the sample, techniques/tools, conditions, and reagents, etc. used have to be the same as those used for determining the level of NKG2C in the test sample. If not, as the skilled person will recognize, the prognostic information will not be accurate.

In one embodiment, the reference control value is taken from a group of samples from a population of healthy subjects. The age and sex distribution of such population have to be similar to the sample of MS patients evaluated.

In one embodiment the *in vitro* method comprises:
(i) determining the genotype of *NKG2C* gene in a test sample, and
(ii) determining the surface expression level of NKG2C protein by NK cells in the test sample, and
wherein if the genotype is hemizygote and the surface expression level of NKG2C protein is lower than the reference control value, it is indicative of higher risk for progression of the disease, that is bad prognosis.

In another embodiment, the sample is total blood.

Advantageously, the method of the invention allows the prognosis using non-invasive techniques because both *NKG2C* genotype and NKG2C expression in NK cells can be analysed in blood.

In order to perform the determination of the NKG2C surface expression level, it is possible to use whole blood or the peripheral blood mononuclear cells isolated from total blood. In the present invention the term "peripheral blood mononuclear cells" or "PBMCs" are any blood cell having a round nucleus (as opposed to a lobed nucleus) such as a lymphocyte, a monocyte or a macrophage. These cells can be extracted from whole blood using routinary procedures. Examples of such procedures are: (a) the treatment with ficoll, a hydrophilic polysaccharide that separates layers of blood, which will separate the blood into a top layer of plasma, followed by a layer of PBMCs and a bottom fraction of polymorphonuclear cells (such as neutrophils and eosinophils) and erythrocytes; and (b) lysing the red blood cells. The skilled person using his general knowledge is able of selecting those appropriate conditions and reactants for isolating PBMCs.

In one embodiment, the genotype is determined by PCR. The conditions for performing the PCR can be determined by the skilled person in the art from the general knowledge and routine tasks. As an example, Miyashita et al. (Miyashita et al., 2004) and Moraru et al. (Moraru 2014) disclose conditions, reagents, and primers for NKG2C genotyping based on PCR.

In another embodiment, the PCR is performed using two pairs of primers SEQ ID NO:1 with SEQ ID NO: 2, and SEQ ID NO: 3 with SEQ ID NO: 4.

In still another embodiment of the method of the first aspect of the invention, the surface expression level of NKG2C protein by NK cells corresponds to the % of NK cells expressing NKG2C surface protein in the sample.

In still another embodiment of the method of the first aspect of the invention, the surface expression level of NKG2C protein by NK cells is determined using an antibody or a fragment thereof which specifically binds to it.

In still another embodiment of the method of the first aspect of the invention, the level of NKG2C surface expression by NK cells corresponds to the % of NK cells expressing NKG2C surface protein in the sample, using an antibody or a fragment thereof which specifically binds to it.

In the present invention, the term "antibody or a fragment thereof with the ability of binding to NKG2C" is to be understood as any immunoglobulin or fragment thereof able to bind the antigen defined by NKG2C. It includes monoclonal and polyclonal antibodies. The term "fragment thereof" encompasses any part of an antibody having the size and conformation suitable to bind an epitope of NKG2C. Suitable fragments include F(ab), F(ab') and Fv. An "epitope" is the part of the antigen being recognized by the immune system (B-cells, T-cells or antibodies).

There are well known means in the state of the art to prepare and characterise antibodies. The methods to generate polyclonal antibodies are well known in the state of the art. Briefly, a polyclonal antibody is prepared by immunising an animal with an immunogenic composition and collecting serum from the immunised animal. A wide range of animal species can be used to produce the antiserum. The animals typically used to produce antiserum can be rabbits, mice, rats, hamsters, guinea pigs or goats.

On the other hand, monoclonal antibodies (MAbs) can be easily prepared using well-known techniques. The procedures for preparing monoclonal antibodies are generally started along the same lines as the preparation of polyclonal antibodies. Animals are injected the antigen as indicated above. The antigen can be mixed with adjuvants, such as Freund's complete or incomplete adjuvant. Vaccination with the same antigen is repeated approximately every two weeks. After immunisation, somatic cells with potential for antibody production are chosen, specifically B lymphocytes (B cells), for use in the protocol for MAb generation. These cells can be obtained from biopsied spleen, lymph nodes or tonsils, or from peripheral blood samples. Antibody-producing B lymphocytes from the immunised animal are then fused with cells from a line of immortal myeloma cells, generally from the same species as the immunised animal. Myeloma cell lines that are adequate for use in fusion procedures for the production of hybridomas are preferably not antibody-producing cells, but have high fusion efficacy and enzyme deficiencies that therefore make them unable to grow in certain culture mediums that only support the growth of the desired fused cells (hybridomas).

In one embodiment, NKG2C is detected using a monoclonal antibody. There are several commercial antibodies, for example, from R&D Systems anti-NKG2C such as monoclonal human NKG2C/CD159c antibodies from R&D Systems (such as the one with catalogue number MAB1381).

In another embodiment, the surface expression level of NKG2C protein is detected by immunostaining.

In the present invention, the term "immunostaining" as used herein refers to a variety of techniques for detecting antigens (usually proteins and peptides) in a sample by exploiting the principle of antibodies binding specifically to said antigens. These antibodies can be monoclonal or polyclonal. Detection of this first or primary antibody can be accomplished in multiple ways: (a) directly labeled using an enzyme or fluorochrome; (b) labeled using a small molecule which interacts with a high affinity binding partner that can be linked to an enzyme or fluorochrome. The biotin-streptavidin is one commonly used high affinity interaction; (c) probed for using a broader species-specific secondary antibody that is labeled using an enzyme, or fluorochrome; and (d) in the case of electron microscopy, antibodies are linked to a heavy metal atom. Illustrative non-limitative examples of immunostaining techniques are: flow cytometry, western blot, and ELISA.

A flow cytometer can be used for the direct analysis of cells expressing one or more specific proteins. Cells are immunostained in solution using methods similar to those used for immunofluorescence, and then analysed by flow cytometry. The immunostaining can be direct (e.g., using a NKG2C/CD159c PE-conjugated antibody (monoclonal mouse IgG1, clone #134591, Catalog number: FAB138P, R&D Systems)) or indirect (e.g., MAB1381, R&D Systems). Flow cytometry has several advantages: the ability to define distinct cell populations by their size and granularity; the capacity to gate out dead cells; improved sensitivity; and multi-colour analysis to measure several antigens simultaneously. It is part of the routine task of the skilled person in the art to determine the specific conditions for analysing a sample by flow cytometry.

Western blotting allows the detection of specific proteins from extracts made from cells or tissues, before or after any purification steps. Proteins are generally separated by size using gel electrophoresis before being transferred to a synthetic membrane via dry, semi-dry, or wet blotting methods. The membrane can then be probed using antibodies using methods similar to immunohistochemistry, but without a need for fixation. Detection is typically performed using peroxidase linked antibodies to catalyse a chemiluminescent reaction. The size separation prior to blotting allows the protein molecular weight to be gauged as compared with known molecular weight markers.

The enzyme-linked immunosorbent assay or ELISA is useful for quantitatively or semi-quantitatively determining protein concentrations from blood plasma, serum or cell/tissue extracts in a multi-well plate format (usually 96-wells per plate). Broadly, proteins in solution are adsorbed to ELISA plates. Antibodies specific for the protein of interest are used to probe the plate. Background is minimised by optimising blocking and washing methods (as for IHC), and specificity is ensured via the presence of positive and negative controls. Detection methods are usually colorimetric or chemiluminescence based.

In one embodiment, the surface expression of NKG2C protein level is determined by flow cytometry.

In another embodiment the present invention provides an *in vitro* method for determining the clinical prognosis of MS in a HCMV seropositive MS patient, the method comprising the step of determining the genotype of *NKG2C* gene in an isolated blood sample from the subject, wherein if the genotype of *NKG2C* gene is hemizygote, it is indicative that the subject has a bad prognosis.

In another embodiment the present invention provides an *in vitro* method for determining the clinical prognosis of MS in a HCMV seropositive MS patient, the method comprising the step of determining the genotype of *NKG2C* gene by PCR in an isolated blood sample from the subject, wherein if the genotype of *NKG2C* gene is hemizygote, it is indicative that the subject has a bad prognosis.

In another embodiment the present invention provides an *in vitro* method for determining the clinical prognosis of MS in a HCMV seropositive MS patient, the method comprising the step of determining the genotype of *NKG2C* gene by PCR using two pair of primers: SEQ ID NO:1 with SEQ ID NO: 2, and SEQ ID NO: 3 with SEQ ID NO: 4, in an isolated blood sample from the subject, wherein if the genotype of *NKG2C* gene is hemizygote, it is indicative that the subject has a bad prognosis.

In another embodiment the *in vitro* method comprises:
(i) determining the genotype of *NKG2C* gene in a blood sample, and
(ii) determining the protein surface expression of NKG2C by NK cells in isolated whole blood or PBMCs test sample, and wherein if the genotype is hemizygote and the surface expression level of NKG2C protein is lower than the reference control value, it is indicative of a high probability of progression of the disease, that is bad prognosis.

In another embodiment the *in vitro* method comprises:
(i) determining the genotype of *NKG2C* gene in the blood test sample performing a PCR, and
(ii) determining the surface expression level of NKG2C protein by NK cells in isolated whole blood or PBMCs test sample and
wherein if the genotype is hemizygote and the surface expression level of NKG2C protein is lower than the reference control value, it is indicative of a high probability of progression of the disease, that is bad prognosis.

In another embodiment the *in vitro* method comprises:
(i) determining the genotype of *NKG2C* gene in the blood sample performing a PCR using the pair of primers: SEQ ID NO:1 with SEQ ID NO: 2, and SEQ ID NO: 3 with SEQ ID NO: 4, and
(ii) determining the surface expression level of NKG2C protein by NK cells in isolated whole blood or PBMCs test sample, and
wherein if the genotype is hemizygote and the surface expression level of NKG2C protein is lower than the reference control value, it is indicative of a high probability of progression of the disease, that is bad prognosis.

In another embodiment the *in vitro* method comprises:
(i) determining the genotype of *NKG2C* gene in the test blood sample, and
(ii) determining the surface expression of NKG2C protein by NK cells in isolated whole blood or PBMCs test sample, using an antibody or a fragment thereof that specifically binds to it,
wherein if the genotype is hemizygote and the surface expression level of NKG2C protein is lower than the reference control value, it is indicative of a high probability of progression of the disease, that is bad prognosis.

In another embodiment the *in vitro* method comprises:
(i) determining the genotype of *NKG2C* gene in the blood sample, and
(ii) determining the surface expression level of NKG2C protein of NKG2C in isolated whole blood or PBMCs test sample by flow cytometry,
wherein if the genotype is hemizygote and the surface expression level of NKG2C protein is lower than the reference control value, it is indicative of a high probability of progression of the disease, that is bad prognosis.

In another embodiment the *in vitro* method comprises:
(i) determining the genotype of *NKG2C* gene in the blood test sample, and
(ii) determining by flow cytometry the surface expression level of NKG2C protein in isolated whole blood or PBMCs test sample using an antibody or a fragment thereof that specifically binds to it,
wherein if the genotype is hemizygote and the surface expression level of NKG2C protein is lower than the reference control value, it is indicative of a high probability of progression of the disease, that is bad prognosis.

In still another embodiment the *in vitro* method comprises:
(i) determining the genotype of *NKG2C* gene in the blood test sample performing a PCR, and
(ii) determining the surface expression level of NKG2C protein in isolated whole blood or PBMCs test sample by flow cytometry, and
wherein if the genotype is hemizygote and the surface expression level of NKG2C protein is lower than the reference control value, it is indicative of a high probability of progression of the disease, that is bad prognosis.

In another embodiment the *in vitro* method comprises:
(i) determining the genotype of *NKG2C* gene in the blood test sample performing a PCR, and
(ii) determining the surface expression level of NKG2C protein in isolated whole blood or PBMCs test sample using an antibody or a fragment thereof that specifically binds to it,
wherein if the genotype is hemizygote and the surface expression level of NKG2C protein is lower than the reference control value, it is indicative of a high probability of progression of the disease, that is bad prognosis.

In another embodiment the *in vitro* method comprises:
(i) determining the genotype of *NKG2C* gene in the blood test sample performing PCR, and
(ii) determining by flow cytometry the surface expression level of NKG2C protein in isolated whole blood or PBMCs test sample using an antibody or a fragment thereof that specifically binds to it,
wherein if the genotype is hemizygote and the surface expression level of NKG2C protein is lower than the reference control value, it is indicative of a high probability of progression of the disease, that is bad prognosis.

In another embodiment the *in vitro* method comprises:
(i) determining the genotype of *NKG2C* gene in the blood test sample performing PCR using two pair of primers: SEQ ID NO:1 with SEQ ID NO: 2, and SEQ ID NO: 3 with SEQ ID NO: 4, and
(ii) determining by flow cytometry the surface expression level of NKG2C protein in isolated whole blood or PBMCs test sample,
wherein if the genotype is hemizygote and the surface expression level of NKG2C protein is lower than the reference control value, it is indicative of a high probability of progression of the disease, that is bad prognosis.

In another embodiment the *in vitro* method comprises:
(i) determining the genotype of *NKG2C* gene in the blood test sample performing PCR using two pair of primers: SEQ ID NO:1 with SEQ ID NO: 2, and SEQ ID NO: 3 with SEQ ID NO: 4, and
(ii) determining the surface expression level of NKG2C protein in isolated whole blood or PBMCs test sample using an antibody or a fragment thereof that specifically binds to it,
wherein if the genotype is hemizygote and the surface expression level of NKG2C protein is lower than the reference control value, it is indicative of a high probability of progression of the disease, that is bad prognosis.

In another embodiment the *in vitro* method comprises:
(i) determining the genotype of *NKG2C* gene in blood test sample performing PCR using two pair of primers: SEQ ID NO:1 with SEQ ID NO: 2, and SEQ ID NO: 3 with SEQ ID NO: 4, and
(ii) determining by flow cytometry the surface expression level of NKG2C protein in isolated whole blood or PBMCs test sample using an antibody or a fragment thereof that specifically binds to it,
wherein if the genotype is hemizygote and the surface expression level of NKG2C protein is lower than the reference control value, it is indicative of a high probability of progression of the disease, that is bad prognosis.

In a second aspect, the present invention provides the use of means for performing the method of the first aspect of the invention.

In another embodiment, the means is an antibody or a fragment thereof which specifically binds to NKG2C or a nucleic acid analysis reagent.

In yet another embodiment, the means forms part of a kit.

The kit may additionally comprise further means (additives, solvents) to visualize the interactions (dipsticks, chemiluminescent reagents, turbidimetric reagents, etc.). Suitable additives, solvents and reagents to visualize the antigen-antibody interaction are disclosed in the examples.

The *in vitro* method of the invention provides prognostic information. In one embodiment, the method of the first aspect of the invention further comprises the steps of (c) collecting the prognostic information, and (d) saving the information in a data carrier.

In the sense of the invention a "data carrier" is to be understood as any means that contain meaningful information data for the prognosis of MS. Examples of data carrier are printed copies of paper with genotype and phenotype of NKG2C determined according to these methods and correlating with the prognosis of the disease. The carrier may also be any entity or device capable of carrying the prognosis data. For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal, which may be conveyed via electrical or optical cable or by radio or other means. When the prognosis data are embodied in a signal that may be conveyed directly by a cable or other device or means, the carrier may be constituted by such cable or other device or means. Other carriers relate to USB devices and computer archives. Examples of suitable data carrier are paper, CDs, USB, computer archives in PCs, or sound registration with the same information.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### EXAMPLES

### 1. PATIENTS AND METHODS

### - Study design and MS population: Cross-sectional study of historical prospective MS cohorts.

A total of 104 Multiple sclerosis (MS) patients were asked to participate in the study after written informed consent. Conventional clinical variables at the time of inclusion were evaluated in all patients (age, sex, ethnicity, age at disease onset, time of MS evolution, MS type (RRMS, SPMS, PPMS), current or previous use of disease-modifying drugs, EDSS (Expanded Disability Status Scale), MSSS (Multiple Sclerosis Severity Score), annualized relapse rate, number of relapses in the previous two years). Clinical variables from MS patients were obtained from clinical databases prospectively gathered following local standards. MS patients were identified based on the following clinical inclusion / exclusion criteria:
Inclusion criteria: 1) MS according to McDonald criteria 2005 or 2010 (Polman 2005, Polman 2011). Benign MS was defined as an EDSS ≤ 3.0 and time of evolution ≥10 years (Correale J et al., 2012). Non-benign MS was defined as an EDSS >3.0 sustained for at least 6 months at any time of disease evolution. 2) Neurological stability for at least 30 days.
Exclusion criteria: 1) Use of lymphocyte depleting or lymphocyte circulationblocking therapies (e.g., fingolimod, mitoxantrone, azathioprine, natalizumab, alemtuzumab) within 24-months previous to study inclusion. The use of interferon-beta was not considered an exclusion criteria since previous studies have not shown modifications in NKG2C protein related to this therapy (Martinez-Rodriguez 2011). 2) Current inclusion in a clinical trial on disease-modifying drugs. 3) Concomitant major disease or pregnancy. 4) Use of corticosteroids in the previous 30 days.

Blood samples from heparinized peripheral blood obtained by venous puncture was fractionated to obtain DNA, HCMV serology, and the analysis of Natural Killer Receptors (NKR) surface expression in peripheral blood mononuclear cells (PBMC) by flow cytometry.

### - NKG2C genotype:

DNA was isolated from total blood using the Puregene, BloodCore Kit B (Qiagen), and *NKG2C* zygosity was assessed by standard single-tube polymerase chain reaction as in Moraru et al (Moraru 2012). Briefly, two previously reported primers (SEQ ID NO: 1 and 2) were specific for the intergenic regions flanking NKG2C and generate a 411-bp amplicon only in carriers of an NKG2C deletion, in whom the two regions come to close proximity; whereas a second primer pair (SEQ ID NO: 3 and 4) amplified, in DNAs having a NKG2C gene, a 201-bp fragment from exon 6 and its 3' untranslated region:
- SEQ ID NO: 1: ACTCGGATTTCTATTTGATGC
- SEQ ID NO: 2: ACAAGTGATGTATAAGAAAAAG
- SEQ ID NO: 3: CAGTGTGGATCTTCAATG
- SEQ ID NO: 4: TTTAGTAATTGTGTGCATCCTA

In each reaction, 100 ng of genomic DNA were amplified in 10 µl of PCR buffer (67 mM Tris-HCl, pH 8.8, 16 mM (NH₄)₂SO₄, 2 mM MgCl₂, 0.01 % Tween-20 and 100 mM deoxyribonucleotide triphosphates) containing 0.3 U of Taq polymerase (Bioline, London, UK). The concentration of each primer was adjusted empirically and may need local re-adjustment so that bands of similar intensities are produced by each primer pair. We have used PTC-200 and PTC-100 (MJR,Watertown, MA) thermocyclers in the following PCR conditions: 2 min at 95 °C, then 10 cycles of 10 s at 95 °C, 20 s at 60 °C and 30 s at 72 °C and 20 cycles of 10 s at 95 °C, 20 s at 56 °C and 30 s at 72 °C.

From every individual, the multiplex reaction always amplifies one or both of the possible amplicons, therefore it does not require an additional primer pair serving as an internal positive control. The amplicons thus generated can then be separated and distinguished, without further manipulation, by their different electrophoretic mobilities in agarose gels 2% agarose. The hemizygotes shown two bands, whereas homozygotes shown one single band.

Three of the subjects were found to have a homozygous deletion of the NKG2C gene, and were excluded from the analysis.

### - NKG2C expression by NK cells:

Peripheral blood mononucleated cell (PBMC) were isolated using a standard Ficoll-Hypaque density gradient centrifugation, followed by tranfer of the layer containing the PBMC, washed twice in PBS 1X, and subsequently cryopreserved. For indirect immunofluorescence staining, cells were labeled at saturating concentrations with the human NKG2C/CD159c antibody (monoclonal mouse IgG2b clone #134522, Catalog Number: MAB1381, R&D Systems). A secondary staining was performed incubating the individual primary antibodies followed by washing and labeling with phycoerythrin PEtagged F(ab')2 rabbit anti-mouse Ig antibodies (Dako Glostrup, Denmark). For NK cell gating, an antibody panel was used including the following antihuman monoclonal antibodies: anti-CD3-PerCP, anti-CD56-APC (Becton Dickinson Bioscience, San Jose, California, USA), anti-CD14-PeCy7 (eBioscience, San Diego, California, USA). Samples were analyzed on an LSR-II flow cytometer (BD Biosciences), defining NK cells as CD3-CD56+ lymphocytes. Flow cytometry data was expressed as the percentage of specifically stained cells, calculating absolute numbers of NKR+ cells based on blood counts obtained in parallel.

### - HCMV serological analysis:

Standard clinical diagnostic tests were used to analyze serum samples for circulating IgG and IgM antibodies against HCMV .

### - Statistical analysis:

Normal Q-Q probability plots assessed normal distribution of continuous variables. Student t-test or Mann-Whitney U-test were used to test the relationship between continuous or dichotomous variables. For dichotomous analysis of NKG2C expression, a NKG2C bright pattern was defined using the mean ± 2SD of the % NKG2C in HCMV seronegative healthy subjects (Muntasell 2013). Kaplan-Meier event-free survival curves was performed, being the endpoint the time to reach an EDSS >3.0. Results were considered significant at the 2-sided level of 0.05.

### RESULTS

### - Clinical variables and HCMV serology:

As detailed in methods, after exclusion of 3 cases *NKG2C* -/-, the remaining 101 MS patients (59 RRMS, 30 SPMS and 12 PPMS) were evaluated based on conventional clinical parameters of disease activity and disability, *NKG2C* genotype and NKG2C expression by NK cells. Serum samples from MS patients and 50 healthy controls were analyzed for HCMV-specific circulating IgG antibodies (MS patients HCMV+, 73 cases (72.3%); controls, 33 cases (66%)) and EBNA-1 titers (MS patients, 100% EBV+; controls, 92.1 %). HCMV seroprevalence was unrelated to MS clinical variables. From the group of MS patients HCMV+, 37 cases had a benign MS. No difference in time of disease evolution was observed between benign and non-benign MS (18.52 ± 8.20, 21.84 ± 11.74, respectively, p 0.160).

### - NKG2C genotype:

From the total of 73 MS patients HCMV+ analyzed, 33 cases were *NKG2C* -/+ and 40 cases were *NKG2C* +/+. Survival analysis according to the *NKG2C* genotype were performed considering time to reach an EDSS >3.0, a representative point for considering benignity in MS, as previously reported (Correale 2012). Patients *NKG2C* -/+ had a significant higher risk to reach an EDSS > 3.0 compared to patients *NKG2C* +/+ (HR 1.930, CI95% 1.006-3.701, p 0.048) (FIG. 1).

### - NKG2C expression by NK cells:

Proportions of NKG2C+ NK cells were lower in HCMV+ MS patients than in HCMV+ controls (14.5% ± 9.5 vs. 22.4% ± 19.1, respectively, p 0.006). Similar findings were obtained when absolute number of peripheral NKG2C+ NK cell was analyzed (MS patients, 33 ± 39 cells/µL vs. controls, 82 ± 94 cells/µL, p 0.001). However, no significant differences were found in univariate analysis for proportions or absolute number of NKG2C+ NK cells according to MS clinical form. As previously reported (Martínez-Rodríguez 2011), NKG2C expression was not modified by interferon-beta therapy, supporting its persistent expression under steady-state conditions (Muntasell 2013).

In order to improve the predictive capability of the *NKG2C* genotype on the risk for MS disability, NKG2C expression by NK cells was introduced in a subsequent survival analysis defining a NKG2C bright or dim expression pattern as previously reported (Muntasell 2013; Martínez-Rodríguez 2013). Among the 33 *NKG2C* -/+ patients, all patients with a NKG2C bright pattern (n=4) had a benign MS compared with 32% of benign MS among patients with NKG2C dim pattern (p 0.019). Analyzing *NKG2C* +/+ patients according to NKG2C bright pattern did not provide any further predictive capability for MS prognosis (NKG2C bright, n=12, 75% benign MS; NKG2C dim, n=25, 48% benign MS; p 0.115). A combine survival analysis according to *NKG2C* -/+, *NKG2C* +/+ and NKG2C bright pattern, considering time to reach an EDSS >3.0 support the value of NKG2C bright after *NKG2C* genotype for further selection of non-benign MS patients (HR 2.348, CI95% 1.200-4.594, p 0.013) (FIG. 2).

### CONCLUSIONS

The clinical course of the disease according to *NKG2C* genotype and NKG2C surface expression by NK cells in MS patients seropositive for HCMV was evaluated. The *NKG2C* gene copy number analysis in HCMV seropositive patients showed that *NKG2C* +/+ patients had a significant delayed time period for reaching an EDSS > 3.0 compared to *NKG2C* -/+ patients. This finding was supported by surface NKG2C expression by NK cells, both findings suggesting that higher NKG2C expression in benign MS is not merely a HCMV-related epiphenomenon but may reflect a direct involvement of the NKG2C+ NK cell subset in MS pathogenesis.

Being the *NKG2C* gene a copy number effect on MS, the effect of this genotype on MS clinical course and predisposition could not be detected in previous genome-wide association studies evaluating genetic risks for MS.

In summary:
1) The analysis of *NKG2C* genotype and NKG2C expression appears a more refined approach than conventional serological tests to assess the putative beneficial effect of the HCMV-host relationship on the clinical course of MS.
*2) NKG2C* hemizygosity and lower NKG2C expression levels in HCMV+ MS patients might be associated with a higher probability of progression, suggesting a protective role of the NKG2C bright NK cell subset.
3) The analysis of *NKG2C* genotype and NKG2C expression may help in the selection of MS patients with a higher risk of progression for whom second- or third-line therapies may be more useful and, subsequently, early implemented.

### REFERENCES CITED IN THE APPLICATION

Burtis C. A. et al., 2008, Chapter 14, section "Statistical Treatment of Reference Values
Correale J, et al., "Benign multiple sclerosis: a new definition of this entity is needed", Mult Scler 2012; 18(2): 210-8.
Polman C. H. et al., "Diagnostic Criteria for Multiple Sclerosis: 2005 Revisions to the "McDonald Criteria", Ann. Neurol., 2005, vol. 58, pages 840-846.
Polman C. H. et al., "Diagnostic Criteria for Multiple Sclerosis: 2010 Revisions to the McDonald Criteria", Ann. Neurol., 2011, vol. 69 (2), pages 292-302.
Martínez-Rodríguez J. E. et al., "Natural killer cell phenotype and clinical response to interferon-beta therapy in multiple sclerosis", Clin Immunol., 2011, vol. 141 (3), pages 348-356.
Martínez-Rodríguez J. E., et al., "Expansion of the NKG2C+ natural killer-cell subset is associated with high-risk carotid atherosclerotic plaques in seropositive patients for human cytomegalovirus", Arterioscler Thromb Vasc Biol 2013; 33 (11): 2653-9.
Miyashita R. et al., "Molecular genetic analyses of human NKG2C (KLRC2) gene deletion", Int. Immunol., 2004, vol. 16 (1), pages 163-168.
Moraru M. et al., "Assessment of copy-number variation in the NKG2C receptor gene in a single-tube and characterization of a reference cell panel, using standard polymerase chain reaction", Tissue Antigens, 2012, vol. 80 (2), pages 184-187.
Muntasell A. et al., "Adaptive reconfiguration of the human NK-cell compartment in response to cytomegalovirus: A different perspective of the host-pathogen interaction", Eur. J. Immunol., 2013, vol. 43(5), pages 1133-1141.

## Claims

1. An *in vitro* method for determining the clinical prognosis in a subject diagnosed of multiple sclerosis (MS), the method comprising the step of determining the genotype of *NKG2C* gene in an isolated test sample from the subject.

2. The in vitro method according to claim 1, wherein the subject is human cytomegalovirus (HCMV) seropositive.

3. The *in vitro* method according to any of the claims 1-2, wherein if the genotype of *NKG2C* gene is hemizygote, it is indicative that the subject has a bad prognosis.

4. The *in vitro* method according to any of the claims 1-3, which further comprises the step of determining the surface expression level of NKG2C protein by NK cells.

5. The *in vitro* method according to claim 4, wherein the determination of the surface expression level of NKG2C protein by NK cells is performed prior, after or simultaneously to the step of genotyping *NKG2C* gene.

6. The *in vitro* method according to any of the claims 4-5, which comprises:
(a) determining the surface expression level of NKG2C protein by NK cells in the test sample of the subject; and
(b) comparing the level determined in step (a) with a reference NKG2C control value,
wherein if the level determined in step (a) is lower than the reference control value, it is indicative that the subject has a bad prognosis.

7. The *in vitro* method according to any of the preceding claims comprising:
(i) determining the genotype of *NKG2C* gene in a test sample, and
(ii) determining the surface expression level of NKG2C protein by NK cells in the test sample,
wherein if the genotype is hemizygote and the surface expression level of NKG2C protein is lower than the reference control value, it is indicative of a higher probability of progression of the disease.

8. The *in vitro* method according to any one of the preceding claims, wherein the test sample is blood.

9. The *in vitro* method according to any one of the preceding claims, wherein NKG2C genotype is determined by PCR.

10. The *in vitro* method according to any one of the claims 4-9, wherein the surface expression level of NKG2C protein is determined using an antibody or a fragment thereof that specifically binds to it.

11. The method according to any one of the claims 1-10, which further comprises the steps of (c) collecting the prognostic information, and (d) saving the information in a data carrier.

12. Use of means for determining the genotype of *NKG2C* in an isolated sample for performing the method of any one of the claims 1 to 10.

13. Use of means for determining the surface expression level of NKG2C protein in an isolated sample for performing the method of any one of the claims 3 to 10.

14. The use of claim 13 wherein the means is an antibody or a fragment thereof which specifically binds to NKG2C or a nucleic acid analysis reagent.

15. The use of any of the claims 12-14, wherein the means forms part of a kit.
